# EUROPEAN PATENT SPECIFICATION

(11) **EP 4 019 066 B1**
(45) Date of publication and mention of the grant of the patent: **31.12.2025**
(21) Application number: 22170978.5
(22) Date of filing: 29.04.2022
(51) Int. Cl.: A61M 5/315, A61M 5/31, A61M 5/50

(54) **CONTAINER WITH PLUNGER AND LOCKING ARRANGEMENT FOR THE PLUNGER**
BEHÄLTER MIT KOLBEN UND VERRIEGELUNGSVORRICHTUNG
RÉCIPIENT DOTÉ D'UN PISTON ET AGENCEMENT DE VERROUILLAGE

(43) Date of publication of application: 29.06.2022
(73) Proprietor: SCHOTT Pharma Schweiz AG, 9000 St. Gallen (CH)
(72) Inventor: Moser, Raymond, 9032 Engelburg (CH)
(74) Representative: Fuchs Patentanwälte Partnerschaft mbB

(56) References cited:
- WO-A1-2014/199478
- US-A1- 2014 350 517
- US-A1- 2020 410 897
- US-B2- 9 180 417
- US-B2- 9 326 914

## Description

The application relates to a container, in particular a syringe or a cartridge, comprising a container body, a plunger and a locking arrangement.

### Background

Medical syringes, in particular when filled with a pharmaceutical and stored at low temperatures, often show large motion of the plunger during the cooling and thawing processes. This motion is mainly due to thermal expansion and contraction of the content of the syringe during the temperature changes and a resultant pressure difference between an inside of the syringe and the environment.

Plunger motion poses a risk to the sterility of the syringe content. For example, a travel length of the plunger due to plunger motion can exceed a tolerance limit of the stopper, which typically forms the distal end of the plunger and which, often having a plurality of elastic sealing ribs, provides a certain tolerance regarding plunger motion without loss of sterility. Sterility of the syringe content is assumed to be lost when a position on the inner wall of the syringe which lies outside the volume enclosed by the stopper in one situation occurs to be enclosed by the stopper, or to lie in a region within a length of the stopper that is considered to be not safely sealed, in another situation, and vice versa. As a consequence, inappropriate choice of the plunger may result in a loss of sterility in later applications.

Choosing a plunger in accordance with the tolerance characteristics dependent on temperature requirements of an intended content of a syringe has proven inconvenient in many situations of supply and production. Furthermore, glass syringes often do not meet the requirements of container closure integrity, CCI, at very low temperatures, for example, around minus 80 degrees Celsius, which are typical storage temperatures of various vaccines.

US 2014/0350517 A1 relates to an injection device with stress protection. US 9,326,914 B2 relates to a device for extracting, storing and/or processing blood or other substances of human or animal origin, and for applying blood compounds or other biological compounds. WO 2014/199478 A1 relates to a suction syringe. US 9,180,417 B2 relates toa combined mixing and discharging device.

Nevertheless, an improved technique is desirable that avoids, or at least mitigates, at least one the aforesaid disadvantages.

### Summary of the Invention

Accordingly, the invention comprises a container according to claim 1.

According to a first aspect, a container in the form of a pharmaceutical syringe or a pharmaceutical cartridge is provided. The container comprises a container body, a plunger comprising a plunger rod which is disposed at least partially within the container body, and a locking arrangement for locking a position of the plunger rod relative to the container body. The locking arrangement is operable between at least a locked position and an unlocked position. The locking arrangement comprises at least one deformable element which is configured for applying a locking force between the plunger rod and an inner wall of the container body when the locking arrangement is in the locked position and for releasing a locking force between the plunger rod and the inner wall of the container body when the locking arrangement is changed from the locked position to the unlocked position.

Thus, plunger motion can be effectively reduced. Moreover, a tightness of the container due to a sealing between the plunger rod and the inner wall of the container can be enhanced. Furthermore, changing the locking arrangement from the locked position to the unlocked position can be reversible. In other words, the locking arrangement can be operable repeatedly. In addition, a force which is to be applied in an operating direction of the plunger rod and which is required for moving the plunger rod when the locking arrangement is in the locked position can be adjusted by suitable adjustment of the locking force.

The locking arrangement is at least essentially attached to the plunger rod. In addition, parts of the locking arrangement can be formed integrally with the plunger rod.

The deformable element can be arranged to extend along an inner periphery of the container body. The inner periphery of the container body can be essentially circular, and the deformable element can have an essentially circular shape. As an alternative, the inner periphery of the container body can be essentially polygonal and the deformable element can have an essentially polygonal shape. As another alternative, the inner periphery of the container body can be essentially oval and the deformable element can have an essentially oval shape The deformable element can further extend adjacent the inner wall of the container body in a longitudinal, in particular an axial, direction and/or in an angular direction of the container body.

The deformable element can be further configured to seal a gap between the inner wall of the container body and the plunger rod when the locking arrangement is in the locked position.

The locking arrangement further comprises a lateral protuberance which extends from a portion of the plunger rod. In addition, the deformable element is arranged to be pressed towards the lateral protuberance when the locking arrangement is in the locked position.

The locking arrangement further comprises a sliding member which is moveable relative to the plunger rod and configured to apply a pressing force on the deformable element towards the lateral protuberance when the locking arrangement is in the locked position.

The lateral protuberance and/or the sliding member are configured to cause deformation and/or displacement of at least a portion of the deformable element towards the inner wall of the container when the locking arrangement is in the locked position. The lateral protuberance can extend from the plunger rod. In addition, the sliding member can comprise a tube in which the plunger rod extends at least partially.

The locking arrangement can further comprise at least one actuating member configured for moving the sliding member towards the deformable element when bringing the locking arrangement to the locked position and/or for releasably holding the locking arrangement in the locked position. The actuating member can comprise at least one wedge.

The actuating member can be arranged between the sliding member and a thumb rest of the plunger rod. The actuating member can be at least partially formed integrally with the sliding member at a proximal end of the sliding member facing a thumb rest of the plunger rod. In addition or as an alternative, the actuating member can be at least partially formed integrally with the thumb rest of the plunger rod, the thumb rest being movable relative to the lateral protuberance.

The locking arrangement can comprise a plurality of actuating members which are usable interchangeably, wherein at least two of the plurality of actuating members differ with respect to a distance by which the sliding member is moved relative to the plunger rod when the actuating member is used and operated, respectively. The plurality of actuating members can comprise a plurality of pins, wherein at least two of the pins differ with respect to a thickness. The plurality of actuating members can be usable interchangeably for adjusting a total break-loose force and/or a total gliding force of the plunger. The total break-loose force and the total gliding force of the plunger can comprise a break-loose force and a gliding force of a stopper attached to the plunger rod, respectively, plus a locking force produced by means of the locking arrangement.

The container may comprise polymer and/or glass. The container can be uncoated or silicone-free coated, in particular at an inner surface of the container, more particularly in an area contacting with the deformable element when the locking arrangement is in the locked position. A material of the deformable element can comprise a silicone. As an alternative, the deformable element can be silicone-free, in particular comprising rubber. In addition, or as an alternative, the deformable element can be silicone oil-free coated, preferably liquid-free coated, or, more preferably, can be uncoated. The plunger can comprise a coating with polytetrafluorethylene, PTFE. The container, including the container body, the plunger and the locking arrangement, can be silicone-free.

The container can be a pharmaceutical container, in particular a syringe or a cartridge.

The container can be pre-filled.

The deformable element can be configured to seal the gap between the inner wall of the container body and the plunger rod against a pressure difference of at least 500 Millibars, preferably at least 600 Millibars, more preferably at least 800 Millibars, at a temperature of minus 80 degrees Celsius.

The locking arrangement can be configured to reduce a motion of the plunger rod relative to the container body to less than 3 Millimeters, preferably less than 2 Millimeters, more preferably less than 1.5 Millimeters, when applying a pressure difference of 800 Millibars at a temperature of minus 80 degrees Celsius.

The deformable element can have a Shore hardness in the range from 25 to 80 Shores A, preferably in the range from 25 to 50 Shores A, more preferably in the range from 30 to 35 Shores A.

The locking arrangement can be configured to apply a locking force in the range from 5 to 15 Newton. The locking force can be defined as a force which is to be applied in an operating direction of the plunger rod and which is required for moving the plunger rod, adjusted for other forces, in particular a break-loose force and/or a gliding force of a stopper attached to the plunger rod.

In particular, the container can be a syringe with a brimful volume in the range from 0.8 to 2 Milliliters, a break-loose force of a stopper attached to the plunger rod can be less than 12 Newton, a gliding force of the stopper can be less than 7 Newton, and a locking force of the locking arrangement can be in the range from 12 to 25 Newton. As an alternative, the container can be a syringe with a brimful volume in the range from 8 to 20 Milliliters, a break-loose force of a stopper attached to the plunger rod can be less than 35 Newton, a gliding force of the stopper can be less than 20 Newton, and a locking force of the locking arrangement can be in the range from 20 to 30 Newton. As another alternative, the container can be a syringe with a brimful volume in the range from 30 to 60 Milliliters, a break-loose force of a stopper attached to the plunger rod can be less than 45 Newton, a gliding force of the stopper can be less than 25 Newton, and a locking force of the locking arrangement can be in the range from 30 to 45 Newton.

A contact length between the deformable element and the inner wall of the container body can be in the range from 0 to 1 Millimeters, preferably 0 Millimeters, when the locking arrangement is in the unlocked position. In addition, or as an alternative, a contact length between the deformable element and the inner wall of the container body can be in the range from 2 to 20 Millimeters, preferably in the range from 3 to 4 Millimeters, when the locking arrangement is in the locked position.

The deformable element can have a single annular contact area with the inner wall of the container body when the locking arrangement is in the locked position.

A contact area of the deformable element with respect to the inner wall of the container body can be larger when the locking arrangement is in the locked position than when the locking arrangement is in the unlocked position.

The container body can be transparent in the region of a contact area of the deformable element with respect to the inner wall of the container body. In addition, a surface of the deformable element as seen through the container body can appear darker when in contact with the contact area than when in a non-contacting state.

The plunger can further comprise a stopper attached to the plunger rod. A material of the stopper and a material of the deformable element can be not the same. The material of the stopper can comprise a rubber, preferably a brombutyl rubber or ethylene propylene diene monomer, EPDM, rubber.

The deformable element can be not in direct contact with the stopper. In this case, the lateral protrusion can extend between the deformable element and the stopper. As an alternative, the deformable element can be in direct contact with the stopper. In this case, the stopper, in particular a proximal portion of the stopper, can at least partially constitute the lateral protrusion.

The following parameters can be fulfilled while the locking arrangement is in the locked position: the brimful volume of the container body is 0.5 to 100 Milliliters, preferably 1 to 20 Milliliters, more preferably 1 to 5 Milliliters; the container is prefilled with a liquid, preferably with 10% to 90%, more preferably 20% to 80%, more preferably 30% to 70%, more preferably 40% to 60% of the brimful volume of the container, more preferably the liquid additionally comprises a sugar or salt; the container comprises a headspace of gas, preferably air, of 0 to 10 Millimeters, preferably 0.5 to 5 Millimeters, more preferably 1 to 4 Millimeters, more preferably 1.5 to 3 Millimeters. In addition, the plunger rod movement can be 3 Millimeters or less, preferably 2 Millimeters or less, more preferably 1 Millimeter or less, more preferably 0.5 Millimeters or less, more preferably 0 Millimeters, after one or both of the following test conditions were applied: the pressure outside the container with regard to the inside was reduced by 100 to 1000 Millibars, preferably 200 to 900 Millibars, more preferably 300 to 800 Millibars, more preferably 400 to 600 Millibars, more preferably 430 Millibars for 1 second to 1 week, preferably 1 minute to 24 hours, more preferably 1 hour to 6 hours, more preferably 180 minutes, or the container was stored at -210°C to 25°C, preferably -120°C to 0°C, more preferably -100°C to -15°C, more preferably -90°C to -40°C, more preferably - 80°C, for 1 second to 2 years, preferably 1 minute to 7 days, more preferably 30 minutes to 24 hours, more preferably 1 hour to 6 hours, more preferably 180 minutes.

### Brief Description of the Drawings

Further details, advantages and objectives of the invention become apparent from the drawings and the detailed description. In the drawings, there is shown:
Fig. 1 a container according to an example;
Figs. 2A and 2B detailed views of the locking arrangement of the container shown in Fig. 1 in different states;
Figs. 3 and 4 detailed views of locking arrangements according to further examples;
Figs. 5 to 6B detailed views of locking arrangements according to further examples in different states;
Fig. 7 a container according to another example, and
Fig. 8 a set of different actuating members.

### Detailed Description

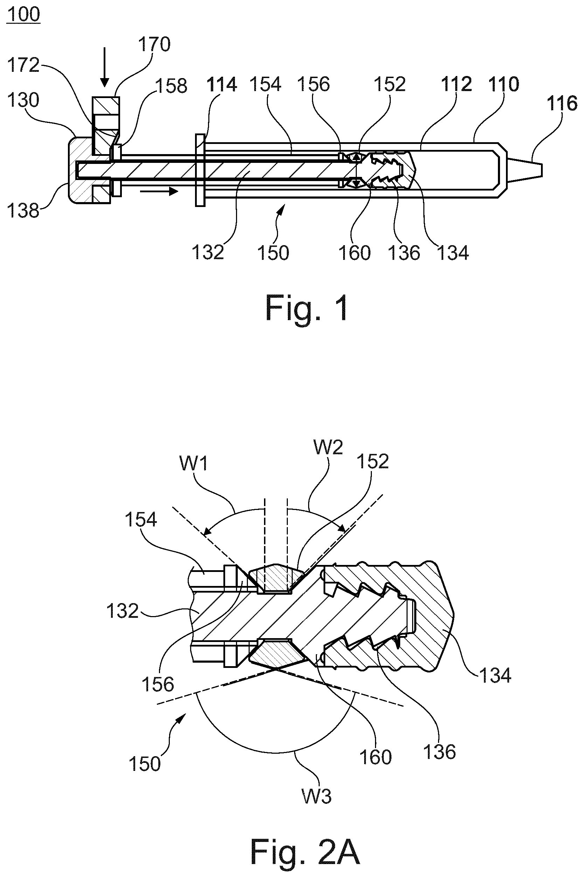

Fig. 1 shows schematically and exemplarily a container 100, in particular a syringe. The container 100 comprises a container body 110 and a plunger 130 which extends partially in the container 110 through an opening 114 at a proximal end of the container 110. The plunger 130 is movable in the container body 110, for example, in order to deliver a content of the container body 110 through a tip 116 of the container body 110.

The plunger 130 comprises a plunger rod 132 and a stopper 134 which is arranged at a distal end of the plunger rod 13. The stopper 134 delimits a variable volume inside the container 110. To this end, the stopper 134 extends adjacent an inner wall 112 of the container 110. The volume in the container 110 which is delimited by the stopper 134 is variable according to a position of the stopper 134, which can be modified through manipulation by means of the plunger rod 132. A thumb rest 138 is provided at a proximal end of the plunger 130. The thumb rest 138 facilitates a delivering of content through the tip 116 by pushing the plunger 130 in an inward direction, especially in a single-handed operation of the container 100. In the shown example, the stopper 134 is attached to the plunger rod 132 by means of a thread 136. The thread 136 facilitates the application of a pulling force on the stopper 134 when pulling the plunger rod 132 in an outward direction. In other examples, other types of connections between the stopper 134 and the plunger rod 132 are used. In some examples, the stopper 134 is formed integrally with the plunger rod 132.

With respect to the aforesaid features and functions, the container 100 essentially resembles a conventional syringe, in particular for pharmaceutical and/or medical use. Furthermore, in some examples the container body 110 comprises polymer and/or glass.

The container 100 further comprises a locking arrangement 150. The locking arrangement 150 is essentially attached to the plunger rod 132 and comprises a deformable element 152, a sliding member 154 having a distal end 156 and a proximal end 158, a lateral protrusion 160 and an actuating member 170.

The sliding member 154 is movable relative to the plunger rod 132 parallel to a longitudinal extension of the plunger rod 132. Moving the sliding member 154 in the distal direction, as indicated in Fig. 1 by the arrow parallel to the plunger rod 132, will cause the distal end 156 of the sliding member 154 to exert a pushing force on the deformable element 152. The deformable element 152 in turn abuts the lateral protrusion 160 extending from the plunger rod 132. As an effect, a compression force will be applied to the deformable element 152.

As shown in Fig. 1, both the distal end 156 of the sliding member 154 and the lateral protrusion have a wedge-shaped profile. As further indicated by the double arrow in Fig. 1, the wedge-shaped profiles of the distal end 156 and the lateral protrusion will cause displacement and/or deformation of the deformable element 152 towards the inner wall 112 of the container 110 under the influence of the longitudinal force applied to the sliding member 154.

By displacing and/or deforming the deformable element 152 towards the inner wall 112, a friction between the deformable element 152 and the inner wall 112 is increased. As a result, motion of the plunger 130 relative to the container body 110 in a longitudinal direction is inhibited.

Furthermore, by displacing and/or deforming the deformable element 152 towards the inner wall 112, a gap between the plunger 130, in particular the plunger rod 132, and the inner wall 112 of the container body 110 becomes sealed. As an effect, a tightness of the container 110 is increased.

In the shown example, each of the container body 110, the sliding member 154, the deformable element 152 and the lateral protrusion 160 has, at least essentially, a radially symmetric shape. For example, the container body 110 is in some implementations at least essentially cylindrical. Moreover, in some examples, the sliding member 154 is essentially tube-shaped, and the deformable element 152 and the lateral protrusion 160 are at least essentially circular and/or ring-shaped. In other examples, each of the container body 110, the sliding member 154, the deformable element 152 and the lateral protrusion 160 has, at least essentially, a polygonal cross-section in a plane that is orthogonal to a longitudinal direction of the cylinder 110. A polygonal cross-section of each of these parts corresponds in some examples to a polygon having an odd number of corners.

The deformable element 152 is essentially ring-shaped. Thus, when the distal end 156 of the sliding member 154 moves towards the lateral protrusion 160, an inner diameter of the deformable element 152 becomes expanded. Accordingly, a profile of the deformable element 152 becomes displaced towards the inner wall 112 of the container 110. Furthermore, the deformable element 152 will be pressed against the inner wall 112 of the container 110, resulting in a deformation of the profile of the deformable element 152.

The deformable element 152 is in some examples made of elastic material, such as an elastic silicone. In addition, in some examples the deformable element 152 has a Shore hardness in the range from 25 to 80 Shores A, preferably in the range from 25 to 50 Shores A, more preferably in the range from 30 to 35 Shores A.

In addition, in some examples a material of the stopper 134 and the material of the deformable element 152 are different. The material of the stopper 134 comprises in some examples a rubber, preferably a brombutyl rubber. Furthermore, in some examples the deformable element 152 and the stopper 134 are not in direct contact.

When a pressing force acting on the deformable element 152, as described above, is released, by moving the sliding member 154 outwardly, the deformable element 152 will return towards its natural state. Accordingly, the deformable element 152 will adopt a state in which it is less displaced towards, and less deformed by, the inner wall 112 of the container body 110. As a consequence, a friction between the deformable element 152 and the inner wall 112 is reduced. A motion of the plunger 130 relative to the container body 110 thus becomes less or not inhibited by the locking arrangement 150. In this manner, the locking arrangement 150 is changed from a locked position to an unlocked position, and vice versa, by moving the sliding member 154 away from, or towards, a position in which the distal end 156 of the sliding member 154 presses on the deformable element 152.

The locking arrangement 150 further comprises an actuating member 170. The actuating member 170 is movable between an unlocked and a locked position. Furthermore, the actuating member 170 is configured to hold the sliding member 154 in a position corresponding to the locked position of the locking arrangement 150, when the actuating member 170 is in its locked position, and to release the sliding member 154 from the position corresponding to the locked position of the locking arrangement 150, when the actuating member 170 is moved from its locked position to its unlocked position.

In some examples, the actuating member 170 is further configured to move the sliding member 154 from a position corresponding to an unlocked position of the locking arrangement 150 to a position corresponding to the locked position of the locking arrangement 150, when the actuating member 170 is moved from its unlocked position to its locked position.

In the example shown in Fig. 1, the actuating member 170 is arranged between the thumb rest 138 and the proximal end 158 of the sliding member 154. Furthermore, in the shown example, the actuating member 170 is formed separate from the plunger 130 and the sliding member 154. In some examples, the actuating member 170 is formed as a separate part which is removable from the plunger 130. In other examples, the actuating member 170 is movably attached to the plunger 130.

In some examples, the actuating member 170 further serves as a seal and/or a tampering evidence of the container 100. For this purpose, the actuating member 170, for example, in the form of a spacer, is attached to the plunger 130 in a position corresponding to a locked position of the locking arrangement 150 and is irreversibly removable from the plunger 130.

Fig. 1 shows the locking arrangement 150 in the unlocked position. As can be seen from Fig. 1, moving the sliding member 154 in an inward direction, corresponding to a locked position of the locking arrangement 150, will enable the insertion of the actuating member 170 between the thumb rest 138 and the proximal end 158 of the sliding member 154. Once inserted, the actuating member 170 will keep the locking arrangement 150 releasably in the locked position.

The actuating member 170 comprises a wedge 172, which is facing the proximal end 158 of the sliding member 154. By means of the wedge 172, when the actuating member 170 is moved into a position between the thumb rest 138 and the sliding member 154, as indicated by the arrow next to the actuating member 170 in Fig. 1, the sliding member 154 will be moved in the inward direction and, in consequence, the locking arrangement 150 will be brought into the locked position. In this way, the wedge 172 facilitates an operation of the locking system 150.

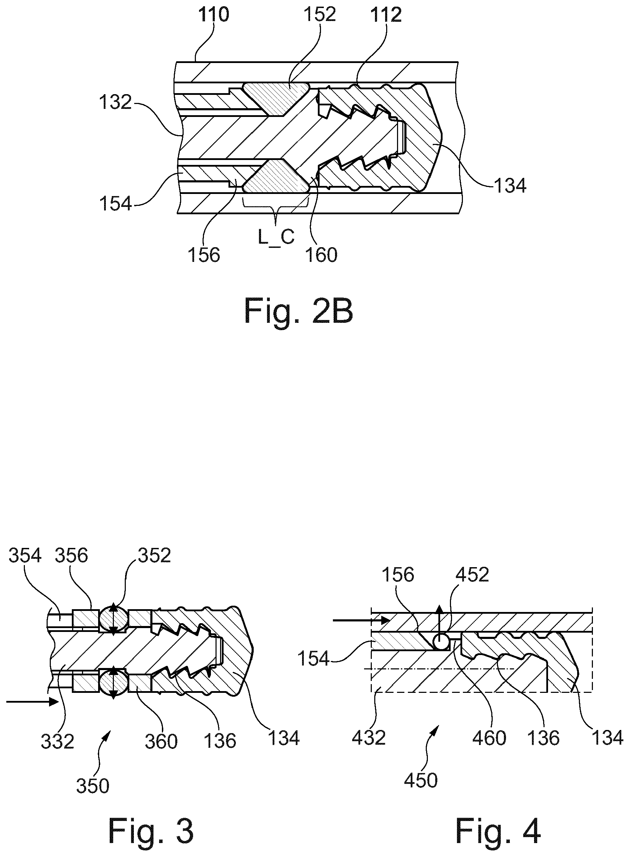

Figs. 2A and 2B show details of the locking arrangement 150 in the unlocked and in the locked position. Identical reference numbers as in Fig. 1 denote identical features. As shown in Fig. 2A, the distal end 156 of the sliding member 154 is angled relative to an orthogonal plane by an angle W1. Moreover, the surface of the lateral protrusion 160 is angled relative to an orthogonal plane by an angle W2. The angles W1, W2 determine an extent of displacement of the deformable element 152 in relation to a length by which the sliding member 154 is moved towards the lateral protrusion 160. In general, the larger the angles W1, W2 are chosen, the less of an operating force needs to be applied on the sliding member 154, at the expense of an increased length by which the sliding member 154 needs to be moved when operating the locking arrangement 150.

Furthermore, the profile of the deformable element 152 is peaked at an outer surface of the deformable element 152 by the angle W3. A peaked profile causes an increased local locking force in the region of the peak, when the locking arrangement 150 is in the locked position. This gives rise to an increased local friction and improved sealing by the deformable element. At the same time, a contact length between the deformable element 152 and the inner wall 112 of the container 110 when the locking arrangement 150 is in the unlocked position is effectively reduced. Furthermore, the angle W3 determines a deformation of the deformable element 152 when the latter is pressed against the inner wall 112 in the locked position of the locking arrangement 112. The larger the angle W3 is chosen, the larger will be a contact length of the deformable element 112 with the inner wall 112 of the container 110 when the locking arrangement 150 is in the locked position. Moreover, a homogeneity of the contact pressure over the contact length between the deformable element 152 and the inner wall 112 can be adjusted by the choice of the angle W3.

Fig. 2B shows the locking arrangement 150 in the locked position. As described above in connection with Fig. 2A, the outer surface of the deformable element 152 is pressed against the inner wall 112 of the container 110, causing deformation of the profile of the deformable element 152. In particular, the peaked outer surface of the deformable element 152 in the relaxed state has been deformed to create a contact length L_C with the inner wall 112 which extends essentially over a total length of the deformable element 152.

In some examples, the container body 110 is uncoated or silicone-free coated in the area which is in contact with the deformable element 152 when the locking arrangement 150 is in the locked position. In addition, in some examples a material of the deformable element 152 comprises a silicone and/or the deformable element 152 is silicone oil-free coated, preferably liquid-free coated, or, more preferably, is uncoated.

Furthermore, in some examples, the container body 110 is transparent in the area which is in contact with the deformable element 152 when the locking arrangement 150 is in the locked position. In addition, a surface of the deformable element 152 has one or more characteristics, such as a surface roughness, which alter when the surface of the deformable element 152 is pressed against the inner wall 112 of the container body 110 in such way that an optical appearance of the deformable element 152 as seen from an outside of the container body 110 through the inner wall 112, for example a brightness in a greyscale image, changes. This facilitates an inspection of the locking state of the locking arrangement 150 by a user.

Fig. 3 shows schematically and exemplarily a detail of a locking arrangement 350 according to another example. Identical reference numerals as in the preceding Figs. 1 to 2B denote identical features. Furthermore, what has been described above regarding the features and functions of the locking arrangement 150 applies analogously to the locking arrangement 350, unless otherwise clear from the following.

In the locking arrangement 350, the lateral protrusion 360 and the distal end 356 of the sliding member 354 are not angled, but they are formed parallel to an orthogonal plane. As an effect, no displacement of the profile of the deformable element 352 towards the inner wall 112 will be caused directly by moving the sliding member 354 towards the lateral protrusion 360, which is attached to the plunger rod 332 adjacent the thread 136. Instead, compression of the profile of the deformable element 352 will occur between the parallel surfaces of the distal end 356 and the lateral protrusion 360. This in turn will lead to a deformation of the profile of the deformable element 352 such that at least an outer portion of the deformable element 352 will be moved towards the inner wall 112, as indicated by the double arrows.

Fig. 4 shows schematically and exemplarily a detail of a locking arrangement 450 according to another example. Identical reference numerals as in the preceding Figs. 1 to 3 denote identical features. Furthermore, what has been described above regarding features and functions of the locking arrangements 150, 350 applies analogously to the locking arrangement 450, unless otherwise clear from Fig. 4 and the following description.

In the locking arrangement 450, the distal end 156 of the sliding member 154 is angled, whereas the lateral protrusion 460, which extends from the plunger rod 432 adjacent the thread 136, is not angled. Furthermore, a profile of the deformable element 452 is essentially circular.

When the sliding member 154 is moved towards the lateral protrusion 460, the angled distal end 156 will cause a displacement of the profile of the deformable element 452 towards the inner wall 112 of the container 110, as indicated by the arrow next to the deformable element 452. At the same time, the circular shape of the profile of the deformable element 452 facilitates a sliding of the deformable element 452 along the lateral protrusion 460.

In the above examples, a lateral protrusion of each of the locking arrangements is formed separate from the stopper 134. However, in other examples of the described locking arrangements, the lateral protrusion is formed integrally with the stopper 134, especially at a proximal end of the stopper 134. In these examples, firm mounting of the stopper 134 to the plunger rod, for example, by means of a thread, is provided to support a pushing force which is applied to the deformable element when the locking arrangement is in the locked position.

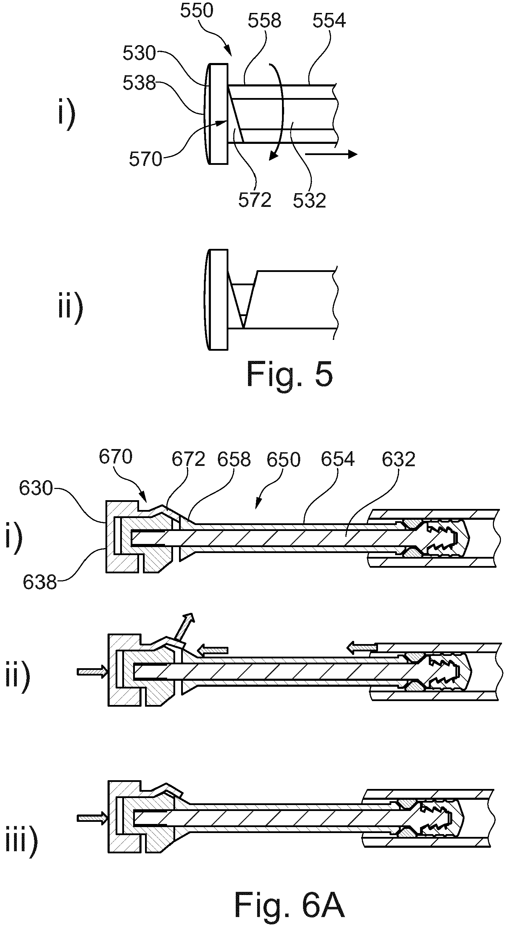

Fig. 5 shows schematically and exemplarily a detail of a locking arrangement 550 according to another example in an unlocked (upper portion of Fig.5) and in a locked state (lower portion of Fig. 5). What has been described above regarding features and functions of the locking arrangements 150, 350, 450 applies analogously to the locking arrangement 550, unless otherwise clear from Fig. 5 and the following description.

In the locking arrangement 550, the at least one actuating member 570 comprises a pair of mutually engaging wedges 558, 572, one of which is integrally formed with the sliding member 554 to constitute a proximal end 558 of the sliding member 554, and the other one is formed at an underside of the thumb rest 538 of the plunger 530.

As indicated in the upper portion of Fig. 5 by the curved arrow, the sliding member 554 can be rotated relative to the plunger rod 532 around its longitudinal axis. The relative position of the wedges 558, 572 is changed in this way, resulting in a motion of the sliding member 554 parallel to its longitudinal axis, i.e., towards or away from the lateral protrusion (not shown) of the locking arrangement 550.

As shown in the lower portion of Fig. 5, a locked position of the locking arrangement 550 corresponds in some examples to a symmetric position of the wedges 558, 572. In this position, no torque on the sliding member 554 results from a reaction force which is exerted on the sliding member 554 by the deformable element (not shown). In addition, a surface friction between the wedges 558, 572 is sufficient in some examples to hold the locking arrangement 550 in the locked position.

In the shown example, the sliding member 554 is rotatable relative to the plunger rod 532 and the thumb rest 538. In other examples, the wedge 572, in some examples together with the thumb rest 538, is rotatable relative to the sliding member 554 and the plunger rod 532.

In a modified example of the locking arrangement 550, the actuating member 570 comprises a pair of engaging threads in place of the wedges 558, 572. In an example, a thread is provided at a distal portion of the sliding member 554 and a corresponding thread engaging with the thread on the sliding member 554 is arranged at an underside of the thumb rest 538 or as a separate part, for example, as a tubular spacer, between the thumb rest 538 and the sliding member 554. Rotation of the engaging threads relative to each other, for example, by rotating the sliding member 554, the tubular spacer, or the thumb rest 538, respectively, will change a distance by which the sliding member 554 is moved in the direction of the deformable element. In some examples, an actuating member 570 comprising a pair of engaging threads is further usable for adjusting, by corresponding rotation of the threads, a locking force which is exerted by the locking arrangement 550.

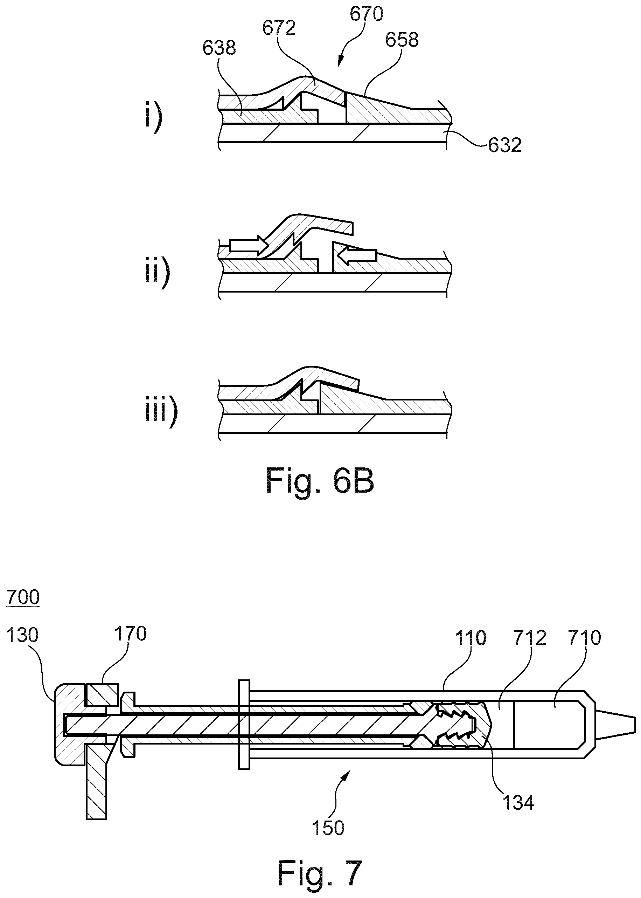

Fig. 6A shows schematically and exemplarily a detail of a locking arrangement 650 according to another example, in a locked state (i), in a released state (ii), and in an unlocked state (iii). What has been described above regarding features and functions of the locking arrangements 150, 350, 450 applies analogously to the locking arrangement 650, unless otherwise clear from Fig. 6 and the following description.

In the locking arrangement 650, the at least one actuating member 670 comprises at least one elastic finger 672, which extends from an underside of the thumb rest 638 of the plunger 630 and which engages with a contour 658 at the proximal end of the sliding member 654.

As shown in the upper portion (i) of Fig. 6A, when the locking arrangement is in the locked position, the elastic finger 672 blocks the contour 658 from moving in the outward direction. In this way, the locking arrangement 650 is held in the locked position.

As shown in the middle portion (ii) of Fig. 6A, when a user pulls the sliding member 654 in the outward direction by manual force, the elastic finger 672 will give way and in this manner will release the sliding member 654 from the blocked state.

As shown in the lower portion (iii) of Fig. 6A, once the sliding member 654 has been moved into a position corresponding to an unlocked position of the locking arrangement 650, the elastic finger 672 will engage with the contour 658 such that the sliding member 654 is held back in the outward position. In this way, the locking arrangement 650 is held in the unlocked state.

Fig. 6B shows further details of the locking arrangement 650. As shown in Fig. 6B, a toothed contour is provided in some examples at the underside of the thumb rest 638. In combination with a toothed contour at the elastic finger 672, it can be provided that unlocking the locking arrangement 650 is irreversible.

Instead of pulling the sliding member 654 in the outward direction, in some examples a user may simply push the thumb rest 638 in the inward direction to unlock the locking arrangement 650. As is apparent from the middle portion (ii) in Fig. 6A, when a user pushes the thumb rest 638 in the inward direction, the plunger 630 may be moved slightly in the inward direction, except for the sliding member 654, which is held back by the deformable element. The slight movement of the plunger 630 relative to the sliding member 654 is enabled by the elastic nature of the elastic finger 672. In turn, the deformation of the elastic finger 672 due to the slight movement of the plunger 630 leads to a releasing of the contour 658 by the elastic finger 672. This operation facilitates a single-handed use of the container and of the locking arrangement 650. In particular, in the case that a user is unaware of the locking arrangement 650 and of its mode of operation, successful application of an injection is still likely, for example, in an emergency situation, since the user is not required to perform any actions different from the use of a conventional syringe, but merely needs to apply an increased pushing force to surmount the resistance exerted by the elastic finger 672.

Fig. 7 shows schematically and exemplarily a container 700 according to another example. Identical reference numerals as in Fig. 1 denote identical features. Furthermore, the above description of Fig. 1 applies correspondingly to the container 700 shown in Fig. 7. A locking arrangement 150 of the container 700 is in the locked position.

The container 700 is pre-filled with a liquid 710. In addition, a gas 712, for example, air, is contained in a headspace of the container 700.

Concerning a motion of the plunger 130, a resultant force acting on the plunger 130 due to thermal expansion and/or contraction of a content of the container 700 depends, among other conditions, on a type and a quantity of the substances 710, 712 contained in the container 700.

In some examples, the deformable element of the locking arrangement 150 is configured to seal the gap between the inner wall of the container body 110 and the plunger 130 against a pressure difference of at least 500 Millibars, preferably at least 600 Millibars, more preferably at least 800 Millibars, at a temperature of minus 80 degrees Celsius.

Furthermore, in some examples, the locking arrangement 150 is configured to reduce a motion of the plunger 130 relative to the container body 110 to less than 3 Millimeters, preferably less than 2 Millimeters, more preferably less than 1.5 Millimeters, more preferably less than 0.5 Millimeters, when applying a pressure difference of 500 Millibars, preferably 600 Millibars, more preferably 800 Millibars, at a temperature of minus 20 degrees Celsius, preferably minus 50 degrees Celsius, more preferably minus 80 degrees Celsius.

The following parameters are fulfilled simultaneously in some examples of the container 700. The brimful volume of the container body 110 is 0.5 to 100 Milliliters, preferably 1 to 20 Milliliters, more preferably 1 to 5 Milliliters. In addition, the container 700 is prefilled with the liquid 710 by 10% to 90%, more preferably 20% to 80%, more preferably 30% to 70%, more preferably 40% to 60% of the brimful volume of the container 700. Preferably, the liquid 710 comprises a sugar or salt. In addition, the container (700) comprises a headspace of gas (712), preferably air, of 0 to 10 Millimeters, preferably 0.5 to 5 Millimeters, more preferably 1 to 4 Millimeters, more preferably 1.5 to 3 Millimeters. In addition, a movement of the plunger rod is 3 Millimeters or less, preferably 2 Millimeters or less, more preferably 1 Millimeter or less, more preferably 0.5 Millimeters or less, more preferably 0 Millimeters, after one or both of the following test conditions were applied.

Test Condition 1: The pressure outside the container 700 with regard to the inside was reduced by 100 to 1000 Millibars, preferably 200 to 900 Millibars, more preferably 300 to 800 Millibars, more preferably 400 to 600 Millibars, more preferably 430 Millibars for 1 second to 1 week, preferably 1 minute to 24 hours, more preferably 1 hour to 6 hours, more preferably 180 minutes.

Test Condition 2: The container 700 was stored at -150°C to 25°C, preferably -120°C to 0°C, more preferably -100°C to -15°C, more preferably -90°C to -40°C, more preferably -80°C, for 1 second to 7 days, preferably 1 minute to 24 hours, more preferably 1 hour to 6 hours, more preferably 180 minutes.

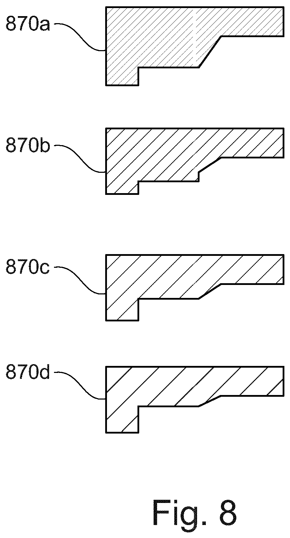

Fig. 8 shows a set of actuating members 870a to 870d. The actuating member 870a to 870d are usable interchangeably, for example, to serve as the actuating member 170, in the form of a pin, in connection with the locking arrangement 150 shown in Figs. 1 and 7. As can be seen in Fig. 8, the actuating members 870a to 870d differ from each other with respect to a thickness of their wedged profile. Accordingly, by selecting a respective one among the actuating members 870a to 870d, a resulting locking force of the locking arrangement can be adjusted. In an example, a total break-loose and gliding force of a plunger as part of a container of the described type is adjusted by corresponding choice of one of the actuating members 870a to 870d.

In the foregoing, examples of locking arrangements have been described in particular connection with a syringe. However, as will be appreciated, some or all of the described features and functions of a locking arrangement can be implemented correspondingly in connection with other types of containers, such as cartridges.

## Claims

1. Container (100; 700) in the form of a pharmaceutical syringe or a pharmaceutical cartridge, comprising:
a container body (110);
a plunger (130; 530; 630) comprising a plunger rod (132; 332; 432; 532; 632), the plunger rod (132; 332; 432; 532; 632) disposed at least partially within the container body (110), and
a locking arrangement (150; 350; 450; 550; 650) for locking a position of the plunger rod (132; 332; 432; 532; 632) relative to the container body (110),
wherein:
the locking arrangement (150; 350; 450; 550; 650) is operable between at least a locked position and an unlocked position, wherein the locking arrangement (150; 350; 450; 550; 650) comprises at least one deformable element (152; 352; 452) which is configured for applying a locking force between the plunger rod (132; 332; 432; 532; 632) and an inner wall (112) of the container body (110) when the locking arrangement (150; 350; 450; 550; 650) is in the locked position and for releasing a locking force between the plunger rod (132; 332; 432; 532; 632) and the inner wall (112) of the container body (110) when the locking arrangement (150; 350; 450; 550; 650) is changed from the locked position to the unlocked position,
the locking arrangement (150; 350; 450; 550; 650) is attached to the plunger rod (132; 332; 432; 532; 632), **characterized in that**
the locking arrangement (150; 350; 450; 550; 650) further comprises:
a lateral protuberance (160; 360; 460) which extends from a portion of the plunger rod (132; 332; 432; 532; 632), and wherein the deformable element (152; 352; 452) is arranged to be pressed towards the lateral protuberance (160; 360; 460) when the locking arrangement (150; 350; 450; 550; 650) is in the locked position, and
a sliding member (154; 354; 554; 654) which is moveable relative to the plunger rod (132; 332; 432; 532; 632) and configured to apply a pressing force on the deformable element (152; 352; 452) towards the lateral protuberance (160; 360; 460) when the locking arrangement (150; 350; 450; 550; 650) is in the locked position, and
the lateral protuberance (160; 360; 460) and/or the sliding member (154; 354; 554; 654) are configured to cause deformation and/or displacement of at least a portion of the deformable element (152; 352; 452) towards the inner wall (112) of the container (110) when the locking arrangement (150; 350; 450; 550; 650) is in the locked position.

2. Container according to claim 1, wherein the deformable element (152; 352; 452) is arranged to extend along an inner periphery of the container body (110), wherein, preferably,
the inner periphery of the container body (110) is essentially circular and the deformable element (152; 352; 452) has an essentially circular shape, or
the inner periphery of the container body (110) is essentially polygonal and the deformable element (152; 352; 452) has an essentially polygonal shape.

3. Container according to any one of the preceding claims, wherein the deformable element (152; 352; 452) is further configured to seal a gap between the inner wall of the container body (110) and the plunger rod (132; 332; 432; 532; 632) when the locking arrangement (150; 350; 450; 550; 650) is in the locked position.

4. Container according to any one of the preceding claims, wherein
the lateral protuberance (160; 360; 460) extends from the plunger rod (132; 332; 432; 532; 632), and the sliding member (154; 354; 554; 654) comprises a tube in which the plunger rod (132; 332; 432; 532; 632) extends at least partially; and/or
the locking arrangement (150; 350; 450; 550; 650) further comprises at least one actuating member (170; 570; 670) configured for moving the sliding member (154; 354; 554; 654) towards the deformable element (152; 352; 452) when bringing the locking arrangement (150; 350; 450; 550; 650) to the locked position and/or for releasably holding the locking arrangement (150; 350; 450; 550; 650) in the locked position.

5. Container according to any one of the preceding claims, wherein
the container body (110) is uncoated or silicone-free coated in an area contacting with the deformable element (152; 352; 452) when the locking arrangement (150; 350; 450; 550; 650) is in the locked position, and/or
a material of the deformable element (152; 352; 452) comprises a silicone, and/or
wherein the deformable element (152; 352; 452) is silicone oil-free coated, preferably liquid-free coated, or, more preferably, is uncoated.

6. Container according to any one of the preceding claims, wherein the container (700) is pre-filled.

7. Container according to any one of the preceding claims in combination with claim 3, wherein the deformable element (152; 352; 452) is configured to seal the gap between the inner wall of the container body (110) and the plunger rod (132; 332; 432; 532; 632) against a pressure difference of at least 500 Millibars, preferably at least 600 Millibars, more preferably at least 800 Millibars, at a temperature of minus 80 degrees Celsius.

8. Container according to any one of the preceding claims, wherein the locking arrangement (150) is configured to reduce a motion of the plunger (130) relative to the container body (110) to less than 3 Millimeters, preferably less than 2 Millimeters, more preferably less than 1.5 Millimeters, more preferably less than 0.5 Millimeters, when applying a pressure difference of 500 Millibars, preferably 600 Millibars, more preferably 800 Millibars, at a temperature of minus 20 degrees Celsius, preferably minus 50 degrees Celsius, more preferably minus 80 degrees Celsius.

9. Container according to any one of the preceding claims, wherein the locking arrangement (150; 350; 450; 550; 650) is configured to apply a locking force in the range from 5 to 15 Newton.

10. Container according to any one of the preceding claims, wherein a contact length (L_C) between the deformable element (152; 352; 452) and the inner wall (112) of the container body (110) is in the range from 0 to 1 Millimeters, preferably 0 Millimeters, when the locking arrangement (150; 350; 450; 550; 650) is in the unlocked position and/or wherein a contact length (L_C) between the deformable element (152; 352; 452) and the inner wall (112) of the container body (110) is in the range from 2 to 20 Millimeters, preferably in the range from 3 to 4 Millimeters, when the locking arrangement (150; 350; 450; 550; 650) is in the locked position.

11. Container according to any one of the preceding claims, wherein the deformable element (152; 352; 452) is not in direct contact with the stopper (134).

12. Container according to any one of the preceding claims, wherein the locking arrangement (150; 350; 450; 550; 650) is in the locked position and the following parameters are fulfilled:
- the brimful volume of the container body (110) is 0.5 to 100 Milliliters, preferably 1 to 20 Milliliters, more preferably 1 to 5 Milliliters;
- the container (700) is prefilled with a liquid (710), preferably with 10% to 90%, more preferably 20% to 80%, more preferably 30% to 70%, more preferably 40% to 60% of the brimful volume of the container (700), more preferably wherein the liquid (710) comprises a sugar or salt,
- the container (700) comprises a headspace of gas (712), preferably air, of 0 to 40 Millimeters, preferably 0.5 to 5 Millimeters, more preferably 1 to 4 Millimeters, more preferably 1.5 to 3 Millimeters;
wherein a movement of the plunger rod is 3 Millimeters or less, preferably 2 Millimeters or less, more preferably 1 Millimeter or less, more preferably 0.5 Millimeters or less, more preferably 0 Millimeters, after one or both of the following test conditions were applied:
- the pressure outside the container (700) with regard to the inside was reduced by 100 to 1000 Millibars, preferably 200 to 900 Millibars, more preferably 300 to 800 Millibars, more preferably 400 to 600 Millibars, more preferably 430 Millibars for 1 second to 1 week, preferably 1 minute to 24 hours, more preferably 1 hour to 6 hours, more preferably 180 minutes;
- the container (700) was stored at -210°C to 25°C, preferably -120°C to 0°C, more preferably -100°C to -15°C, more preferably -90°C to -40°C, more preferably - 80°C, for 1 second to 7 days, preferably 1 minute to 24 hours, more preferably 1 hour to 6 hours, more preferably 180 minutes.

## Patentansprüche

1. Behälter (100; 700) in der Form einer pharmazeutischen Spritze oder einer pharmazeutischen Kartusche, aufweisend:
einen Behälterkörper (110);
einen Kolben (130; 530; 630) mit einer Kolbenstange (132; 332; 432; 532; 632), wobei die Kolbenstange (132; 332; 432; 532; 632) zumindest teilweise innerhalb des Behälterkörpers (110) angeordnet ist; und
eine Verriegelungsanordnung (150; 350; 450; 550; 650) zum Verriegeln einer Position der Kolbenstange (132; 332; 432; 532; 632) relativ zum Behälterkörper (110), wobei:
die Verriegelungsanordnung (150; 350; 450; 550; 650) zwischen mindestens einer verriegelten Position und einer entriegelten Position betätigbar ist, wobei die Verriegelungsanordnung (150; 350; 450; 550; 650) mindestens ein verformbares Element (152; 352; 452) aufweist, das dafür konfiguriert ist, eine Verriegelungskraft zwischen der Kolbenstange (132; 332; 432; 532; 632) und einer Innenwand (112) des Behälterkörpers (110) auszuüben, wenn sich die Verriegelungsanordnung (150; 350; 450; 550; 650) in der verriegelten Position befindet, und eine Verriegelungskraft zwischen der Kolbenstange (132; 332; 432; 532; 632) und der Innenwand (112) des Behälterkörpers (110) freizugeben, wenn die Verriegelungsanordnung (150; 350; 450; 550; 650) von der verriegelten Position in die entriegelte Position gebracht wird, wobei
die Verriegelungsanordnung (150; 350; 450; 550; 650) an der Kolbenstange (132; 332; 432; 532; 632) befestigt ist,
**dadurch gekennzeichnet, dass**
die Verriegelungsanordnung (150; 350; 450; 550; 650) ferner aufweist:
einen sich von einem Abschnitt der Kolbenstange (132; 332; 432; 532; 632) erstreckenden seitlichen Vorsprung (160; 360; 460), wobei das verformbare Element (152; 352; 452) derart angeordnet ist, dass es in Richtung zum seitlichen Vorsprung (160; 360; 460) gedrückt wird, wenn sich die Verriegelungsanordnung (150; 350; 450; 550; 650) in der verriegelten Position befindet; und
ein Gleitelement (154; 354; 554; 654), das relativ zur Kolbenstange (132; 332; 432; 532; 632) bewegbar und dafür konfiguriert ist, eine Drucckraft auf das verformbare Element (152; 352; 452) in Richtung zum seitlichen Vorsprung (160; 360; 460) auszuüben, wenn sich die Verriegelungsanordnung (150; 350; 450; 550; 650) in der verriegelten Position befindet, und
wobei der seitliche Vorsprung (160; 360; 460) und/oder das Gleitelement (154; 354; 554; 654) dafür konfiguriert sind, eine Verformung und/oder einen Versatz mindestens eines Abschnitts des verformbaren Elements (152; 352; 452) in Richtung zur Innenwand (112) des Behälters (110) zu bewirken, wenn sich die Verriegelungsanordnung (150; 350; 450; 550; 650) in der verriegelten Position befindet.

2. Behälter nach Anspruch 1, wobei das verformbare Element (152; 352; 452) derart angeordnet ist, dass es sich entlang eines Innenumfangs des Behälterkörpers (110) erstreckt, wobei vorzugsweise
der Innenumfang des Behälterkörpers (110) im Wesentlichen kreisförmig ist und das verformbare Element (152; 352; 452) eine im Wesentlichen kreisförmige Form aufweist, oder
der Innenumfang des Behälterkörpers (110) im Wesentlichen polygonal ist und das verformbare Element (152; 352; 452) eine im Wesentlichen polygonale Form aufweist.

3. Behälter nach einem der vorhergehenden Ansprüche, wobei das verformbare Element (152; 352; 452) ferner dafür konfiguriert ist, einen Spalt zwischen der Innenwand des Behälterkörpers (110) und der Kolbenstange (132; 332; 432; 532; 632) abzudichten, wenn sich die Verriegelungsanordnung (150; 350; 450; 550; 650) in der verriegelten Position befindet.

4. Behälter nach einem der vorhergehenden Ansprüche, wobei
der seitliche Vorsprung (160; 360; 460) sich von der Kolbenstange (132; 332; 432; 532; 632) erstreckt und das Gleitelement (154; 354; 554; 654) ein Rohr aufweist, in dem sich die Kolbenstange (132; 332; 432; 532; 632) zumindest teilweise erstreckt, und/oder
die Verriegelungsanordnung (150; 350; 450; 550; 650) ferner mindestens ein Betätigungselement (170; 570; 670) aufweist, das dafür konfiguriert ist, das Gleitelement (154; 354; 554; 654) in Richtung zum verformbaren Element (152; 352; 452) zu bewegen, wenn die Verriegelungsanordnung (150; 350; 450; 550; 650) in die verriegelte Position gebracht wird, und/oder die Verriegelungsanordnung (150; 350; 450; 550; 650) lösbar in der verriegelten Position zu halten.

5. Behälter nach einem der vorhergehenden Ansprüche, wobei
der Behälterkörper (110) in einem Bereich, der mit dem verformbaren Element (152; 352; 452) in Kontakt steht, wenn sich die Verriegelungsanordnung (150; 350; 450; 550; 650) in der verriegelten Position befindet, unbeschichtet oder silikonfrei beschichtet ist, und/oder
ein Material des verformbaren Elements (152; 352; 452) ein Silikon aufweist, und/oder
wobei das verformbare Element (152; 352; 452) silikonölfrei beschichtet, vorzugsweise flüssigkeitsfrei beschichtet oder noch bevorzugter unbeschichtet ist.

6. Behälter nach einem der vorhergehenden Ansprüche, wobei der Behälter (700) vorgefüllt ist.

7. Behälter nach einem der vorhergehenden Ansprüche in Kombination mit Anspruch 3, wobei das verformbare Element (152; 352; 452) dafür konfiguriert ist, den Spalt zwischen der Innenwand des Behälterkörpers (110) und der Kolbenstange (132; 332; 432; 532; 632) gegen einen Druckunterschied von mindestens 500 Millibar, vorzugsweise mindestens 600 Millibar, bevorzugter mindestens 800 Millibar, bei einer Temperatur von minus 80 Grad Celsius abzudichten.

8. Behälter nach einem der vorhergehenden Ansprüche, wobei die Verriegelungsanordnung (150) dafür konfiguriert ist, eine Bewegung des Kolbens (130) relativ zum Behälterkörper (110) auf weniger als 3 Millimeter, vorzugsweise weniger als 2 Millimeter, bevorzugter weniger als 1,5 Millimeter, noch bevorzugter weniger als 0,5 Millimeter, zu reduzieren, wenn ein Druckunterschied von 500 Millibar, vorzugsweise 600 Millibar, bevorzugter 800 Millibar, bei einer Temperatur von minus 20 Grad Celsius, vorzugsweise minus 50 Grad Celsius, bevorzugter minus 80 Grad Celsius, ausgeübt wird.

9. Behälter nach einem der vorhergehenden Ansprüche, wobei die Verriegelungsanordnung (150; 350; 450; 550; 650) dafür konfiguriert ist, eine Verriegelungskraft im Bereich von 5 bis 15 Newton auszuüben.

10. Behälter nach einem der vorhergehenden Ansprüche, wobei eine Kontaktlänge (L_C) zwischen dem verformbaren Element (152; 352; 452) und der Innenwand (112) des Behälterkörpers (110) im Bereich von 0 bis 1 Millimeter liegt und vorzugsweise 0 Millimeter beträgt, wenn die Verriegelungsanordnung (150; 350; 450; 550; 650) sich in der entriegelten Position befindet, und/oder wobei eine Kontaktlänge (L_C) zwischen dem verformbaren Element (152; 352; 452) und der Innenwand (112) des Behälterkörpers (110) im Bereich von 2 bis 20 Millimetern, vorzugsweise im Bereich von 3 bis 4 Millimetern, liegt, wenn sich die Verriegelungsanordnung (150; 350; 450; 550; 650) in der verriegelten Position befindet.

11. Behälter nach einem der vorhergehenden Ansprüche, wobei das verformbare Element (152; 352; 452) nicht in direktem Kontakt mit einem Stopfen (134) steht.

12. Behälter nach einem der vorhergehenden Ansprüche, wobei sich die Verriegelungsanordnung (150; 350; 450; 550; 650) in der verriegelten Position befindet und die folgenden Parameter erfüllt sind:
das randvolle Volumen des Behälterkörpers (110) beträgt 0,5 bis 100 Milliliter, vorzugsweise 1 bis 20 Milliliter, bevorzugter 1 bis 5 Milliliter;
der Behälter (700) ist mit einer Flüssigkeit (710) vorgefüllt, vorzugsweise mit 10% bis 90%, bevorzugter 20% bis 80%, noch bevorzugter 30% bis 70%, noch bevorzugter 40% bis 60% des randvollen Volumens des Behälters (700), wobei die Flüssigkeit (710) noch bevorzugter einen Zucker oder ein Salz enthält;
der Behälter (700) weist einen Kopfraum mit Gas (712), vorzugsweise Luft, von 0 bis 40 Millimetern, vorzugsweise 0,5 bis 5 Millimetern, bevorzugter 1 bis 4 Millimetern, noch bevorzugter 1,5 bis 3 Millimetern, auf,
wobei eine Bewegung der Kolbenstange 3 Millimeter oder weniger, vorzugsweise 2 Millimeter oder weniger, bevorzugter 1 Millimeter oder weniger, noch bevorzugter 0,5 Millimeter oder weniger, noch bevorzugter 0 Millimeter, beträgt, nachdem eine oder beide der folgenden Testbedingungen angewendet wurden:
der Druck außerhalb des Behälters (700) wurde bezogen auf den Innenraum um 100 bis 1000 Millibar, vorzugsweise 200 bis 900 Millibar, bevorzugter 300 bis 800 Millibar, noch bevorzugter 400 bis 600 Millibar, noch bevorzugter 430 Millibar für 1 Sekunde bis 1 Woche, vorzugsweise 1 Minute bis 24 Stunden, bevorzugter 1 Stunde bis 6 Stunden, noch bevorzugter 180 Minuten, reduziert;
der Behälter (700) wurde bei -210°C bis 25°C, vorzugsweise -120°C bis 0°C, bevorzugter -100°C bis -15°C, noch bevorzugter -90°C bis -40°C, noch bevorzugter 80°C, für 1 Sekunde bis 7 Tage, vorzugsweise 1 Minute bis 24 Stunden, bevorzugter 1 Stunde bis 6 Stunden, noch bevorzugter 180 Minuten, gelagert.

## Revendications

1. Récipient (100 ; 700) sous la forme d'une seringue pharmaceutique ou d'une cartouche pharmaceutique, comprenant :
un corps de récipient (110) ;
un piston (130 ; 530 ; 630) comprenant une tige de piston (132 ; 332 ; 432 ; 532 ; 632), la tige de piston (132 ; 332 ; 432 ; 532 ; 632) étant disposée au moins partiellement à l'intérieur du corps de récipient (110), et
un dispositif de verrouillage (150 ; 350 ; 450 ; 550 ; 650) pour verrouiller une position de la tige de piston (132 ; 332 ; 432 ; 532 ; 632) par rapport au corps de récipient (110),
dans lequel :
le dispositif de verrouillage (150 ; 350 ; 450 ; 550 ; 650) peut fonctionner entre au moins une position verrouillée et une position déverrouillée, dans lequel le dispositif de verrouillage (150 ; 350 ; 450 ; 550 ; 650) comprend au moins un élément déformable (152 ; 352 ; 452) qui est configuré pour appliquer une force de verrouillage entre la tige de piston (132 ; 332 ; 432 ; 532 ; 632) et une paroi intérieure (112) du corps de récipient (110) lorsque le dispositif de verrouillage (150 ; 350 ; 450 ; 550 ; 650) est en position verrouillée et pour relâcher une force de verrouillage entre la tige de piston (132 ; 332 ; 432 ; 532 ; 632) et la paroi interne (112) du corps de récipient (110) lorsque le dispositif de verrouillage (150 ; 350 ; 450 ; 550 ; 650) est passé de la position verrouillée à la position déverrouillée,
le dispositif de verrouillage (150 ; 350 ; 450 ; 550 ; 650) est fixé à la tige de piston (132 ; 332 ; 432 ; 532 ; 632),
**caractérisé en ce que** le dispositif de verrouillage (150 ; 350 ; 450 ; 550 ; 650) comprend en outre :
une protubérance latérale (160 ; 360 ; 460) qui s'étend à partir d'une partie de la tige de piston (132 ; 332 ; 432 ; 532 ; 632), et dans lequel l'élément déformable (152 ; 352 ; 452) est agencé pour être pressé vers la protubérance latérale (160 ; 360 ; 460) lorsque le dispositif de verrouillage (150 ; 350 ; 450 ; 550 ; 650) est dans la position verrouillée, et
un élément coulissant (154 ; 354 ; 554 ; 654) qui est mobile par rapport à la tige de piston (132 ; 332 ; 432 ; 532 ; 632) et configuré pour appliquer une force de pression sur l'élément déformable (152 ; 352 ; 452) vers la protubérance latérale (160 ; 360 ; 460) lorsque le dispositif de verrouillage (150 ; 350 ; 450 ; 550 ; 650) est dans la position verrouillée, et la protubérance latérale (160 ; 360 ; 460) et/ou l'élément coulissant (154 ; 354 ; 554 ; 654) sont configurés pour provoquer une déformation et/ou un déplacement d'au moins une partie de l'élément déformable (152 ; 352 ; 452) vers la paroi intérieure (112) du récipient (110) lorsque le dispositif de verrouillage (150 ; 350 ; 450 ; 550 ; 650) est dans la position verrouillée.

2. Récipient selon la revendication 1, dans lequel l'élément déformable (152 ; 352 ; 452) est agencé pour s'étendre le long d'une périphérie intérieure du corps de récipient (110), dans lequel, de préférence,
la périphérie intérieure du corps de récipient (110) est essentiellement circulaire et l'élément déformable (152 ; 352 ; 452) a une forme essentiellement circulaire, ou
la périphérie intérieure du corps de récipient (110) est essentiellement polygonale et l'élément déformable (152 ; 352 ; 452) a une forme essentiellement polygonale.

3. Récipient selon l'une quelconque des revendications précédentes, dans lequel l'élément déformable (152 ; 352 ; 452) est en outre configuré pour sceller un espace entre la paroi intérieure du corps de récipient (110) et la tige de piston (132 ; 332 ; 432 ; 532 ; 632) lorsque le dispositif de verrouillage (150 ; 350 ; 450 ; 550 ; 650) est dans la position verrouillée.

4. Récipient selon l'une quelconque des revendications précédentes, dans lequel
la protubérance latérale (160 ; 360 ; 460) s'étend à partir de la tige de piston (132 ; 332 ; 432 ; 532 ; 632), et l'élément coulissant (154 ; 354 ; 554 ; 654) comprend un tube dans lequel la tige de piston (132 ; 332 ; 432 ; 532 ; 632) s'étend au moins partiellement ; et/ou
le dispositif de verrouillage (150 ; 350 ; 450 ; 550 ; 650) comprend en outre au moins un élément d'actionnement (170 ; 570 ; 670) configuré pour déplacer l'élément coulissant (154 ; 354 ; 554 ; 654) vers l'élément déformable (152 ; 352 ; 452) lors de l'amenée du dispositif de verrouillage (150 ; 350 ; 450 ; 550 ; 650) dans la position verrouillée et/ou pour maintenir de manière libérable le dispositif de verrouillage (150 ; 350 ; 450 ; 550 ; 650) dans la position verrouillée.

5. Récipient selon l'une quelconque des revendications précédentes, dans lequel
le corps de récipient (110) n'est pas revêtu ou est revêtu sans silicone dans une zone en contact avec l'élément déformable (152 ; 352 ; 452) lorsque le dispositif de verrouillage (150 ; 350 ; 450 ; 550 ; 650) est dans la position verrouillée, et/ou
un matériau de l'élément déformable (152 ; 352 ; 452) comprend une silicone, et/ou dans lequel l'élément déformable (152 ; 352 ; 452) est revêtu sans huile de silicone, de préférence revêtu sans liquide, ou, de manière plus préférentielle, n'est pas revêtu.

6. Récipient selon l'une quelconque des revendications précédentes, dans lequel le récipient (700) est prérempli.

7. Récipient selon l'une quelconque des revendications précédentes en combinaison avec la revendication 3, dans lequel l'élément déformable (152 ; 352 ; 452) est configuré pour sceller l'espace entre la paroi intérieure du corps de récipient (110) et la tige de piston (132 ; 332 ; 432 ; 532 ; 632) contre une différence de pression d'au moins 500 millibars, de préférence d'au moins 600 millibars, plus préférablement d'au moins 800 millibars, à une température de moins 80 degrés Celsius.

8. Récipient selon l'une quelconque des revendications précédentes, dans lequel le dispositif de verrouillage (150) est configuré pour réduire un mouvement du piston (130) par rapport au corps de récipient (110) à moins de 3 millimètres, de préférence moins de 2 millimètres, plus préférablement moins de 1,5 millimètre, de préférence moins de 0,5 millimètre, lorsqu'une différence de pression de 500 millibars, de préférence 600 millibars, plus préférentiellement 800 millibars, est appliquée à une température de moins 20 degrés Celsius, de préférence moins 50 degrés Celsius, plus préférentiellement moins 80 degrés Celsius.

9. Récipient selon l'une quelconque des revendications précédentes, dans lequel le dispositif de verrouillage (150 ; 350 ; 450 ; 550 ; 650) est configuré pour appliquer une force de verrouillage comprise entre 5 et 15 newtons.

10. Récipient selon l'une quelconque des revendications précédentes, dans lequel une longueur de contact (L_C) entre l'élément déformable (152 ; 352 ; 452) et la paroi intérieure (112) du corps de récipient (110) est comprise entre 0 et 1 millimètre, de préférence 0 millimètre, lorsque le dispositif de verrouillage (150 ; 350 ; 450 ; 550 ; 650) est dans la position déverrouillée et/ou dans lequel une longueur de contact (L_C) entre l'élément déformable (152 ; 352 ; 452) et la paroi intérieure (112) du corps de récipient (110) est comprise entre 2 et 20 millimètres, de préférence entre 3 et 4 millimètres, lorsque le dispositif de verrouillage (150 ; 350 ; 450 ; 550 ; 650) est dans la position verrouillée.

11. Récipient selon l'une quelconque des revendications précédentes, dans lequel l'élément déformable (152; 352 ; 452) n'est pas en contact direct avec le bouchon (134).

12. Récipient selon l'une quelconque des revendications précédentes, dans lequel le dispositif de verrouillage (150 ; 350 ; 450 ; 550 ; 650) est dans la position verrouillée et les paramètres suivants sont remplis :
- le volume à ras bord du corps de récipient (110) est compris entre 0,5 et 100 millilitres, de préférence entre 1 et 20 millilitres, et plus préférablement encore entre 1 et 5 millilitres ;
- le récipient (700) est prérempli d'un liquide (710), de préférence à raison de 10 % à 90 %, de préférence de 20 % à 80 %, de préférence de 30 % à 70 %, de préférence de 40 % à 60 % du volume à ras bord du récipient (700), plus préférentiellement, le liquide (710) comprend un sucre ou un sel,
- le récipient (700) comprend un espace libre rempli de gaz (712), de préférence de l'air, de 0 à 40 millimètres, de préférence de 0,5 à 5 millimètres, plus préférablement de 1 à 4 millimètres, plus préférablement de 1,5 à 3 millimètres ;
dans lequel un mouvement de la tige de piston est de 3 millimètres ou moins, de préférence de 2 millimètres ou moins, plus préférentiellement 1 millimètre ou moins, plus préférentiellement 0,5 millimètre ou moins, plus préférentiellement 0 millimètre, après que l'une ou les deux conditions d'essai suivantes ont été appliquées :
- la pression à l'extérieur du récipient (700) par rapport à l'intérieur a été réduite de 100 à 1000 millibars, de préférence de 200 à 900 millibars, plus préférentiellement de 300 à 800 millibars, plus préférentiellement de 400 à 600 millibars, plus préférentiellement de 430 millibars pendant 1 seconde à 1 semaine, de préférence de 1 minute à 24 heures, plus préférentiellement de 1 heure à 6 heures, plus préférentiellement pendant 180 minutes ;
- le récipient (700) a été stocké à une température comprise entre -210 °C et 25 °C, de préférence - entre -120 °C et 0 °C, plus préférentiellement entre -100 °C et -15 °C, plus préférentiellement entre -90 °C et -40 °C, plus préférentiellement à -80 °C, pendant 1 seconde à 7 jours, de préférence de 1 minute à 24 heures, plus préférentiellement de 1 heure à 6 heures, plus préférentiellement pendant 180 minutes.
